# EUROPEAN PATENT SPECIFICATION

(11) **EP 0 479 442 B1**
(45) Date of publication and mention of the grant of the patent: **18.06.1997**
(21) Application number: 91308368.9
(22) Date of filing: 12.09.1991
(51) Int. Cl.: A61F 13/15, D04H 13/00

(54) **Process and apparatus for forming a composite product**
Verfahren und Vorrichtung zur Herstellung eines zusammengesetzten Produktes
Procédé et dispositif de fabrication d'un produit composite

(30) Priority: 12.09.1990 ZA 907272; 12.09.1990 ZA 907273
(43) Date of publication of application: 08.04.1992
(73) Proprietor: McNEIL-PPC, INC., New Brunswick, New Jersey 08903 (US)
(72) Inventor: Oatley, John Albert, East London, Cape Province (ZA); Bailey, Alan Steven, Gonubie, Cape Province (ZA)
(74) Representative: Fisher, Adrian John

(56) References cited:
- FR-A- 2 521 003
- GB-A- 1 228 877
- US-A- 3 882 216
- US-A- 4 761 258

## Description

This invention relates to a method and apparatus for forming a composite product, and to a composite product so formed.

Absorbent pads for use in absorbing body fluids are well-known. Such pads include baby diapers, sanitary napkins, incontinence pads, wound dressings, breast pads for nursing mothers and the like. They are commonly made of absorbent cellulosic pulp fibres in the form of a batt or wad sandwiched between at least two layers of material. One layer is fluid-permeable and forms the layer adapted to face the wearer's body. The other layer conveniently is liquid-impervious and forms the side which faces away from the person's body and protects the person's clothing from soiling. This invention thus more particularly relates to a process and apparatus for forming a composite product suitable for use as such an absorbent pad or as a component for such a pad.

FR-A-2521003 discloses a method of manufacturing sanitary napkins wherein a fibre loaded airstream is blown against the surface of a rotating drum coated with an air permeable sheet, and the resulting web is subsequently compressed.

GB-A-1228877 discloses a method for continuously making fibrous sheets comprising forming a fibrous layer on a carrier, and subsequently applying a binder to bind the fibrous layer to the carrier.

US-A-3882216 discloses a method of forming an absorptive pad of cellulose fibre material, the method comprising the steps recited in the preamble of claim 1.

According to the invention there is provided a method of manufacturing a composite product, which includes
providing a discrete wad of finely divided material on a carrier;
stabilizing the wad by bonding at least some of the material thereof together; and
bonding at least some of the material in the wad to the carrier by means of thermoplastic particles or a foamed binder.

Further according to the invention there is provided an apparatus for manufacturing a composite product having a wad of finely divided material on a carrier according to US-A-3882216, which includes
a stabilizing and bonding means for stabilizing the wad by bonding at least some of the material thereof together and to the carrier as defined in claim 17.

The carrier may be larger than the wad and that portion of the carrier to which the wad is bonded may be parted from the rest of the carrier by a suitable parting means.

The carrier is fluid permeable and the wad is then formed thereon by a suitable forming means. The carrier may particularly be a non-woven fabric.
Preferably, the carrier is sheet-like and a plurality of discrete wads are formed thereon. These wads are stabilized and bonded to the carrier and the carrier is parted to separate each wad and its associated portion of the carrier from the others. The carrier is parted a predetermined distance from the edge of each wad to provide a borderaround each wad. The apparatus may include a carrier supply means for supplying the carrier from a roll or bobbin, and the wads may be formed thereon and bonded thereto in a continuous manner.

The wad is formed by generating a pressure differential across the carrier, defining an area on the carrier through which a transporting fluid may pass, as a result of the pressure differential, by means of a former; and introducing the finely divided material into the fluid such that the finely divided material is deposited on the carrier in the area defined by the former. There may be a high pressure on one side of the carrier and a low pressure on the other side, the finely divided material being introduced on the high pressure side, and the former being located on the high pressure side of the carrier. Alternatively, the finely divided material may be introduced on the high pressure side and the former may be located on the low pressure side of the carrier.

It will accordingly be appreciated that the forming means includes a pressure differential generating means for generating a pressure differential across the carrier;
a former for defining the desired area on the carrier through which a transporting fluid may pass; and
an introducing means located on a side of the carrier which is, in use, at a higher pressure, for introducing the finely divided material, in use, into a stream of the transporting fluid to be deposited on the carrier in the defined area. Further, the former and the introducing means may be on the same side of the carrier, or on opposite sides.

The carrier may be supported by a fluid permeable support member when the wad is formed thereon and when the wad is stabilized and bonded thereto.

The wad is stabilized and bonded to the carrier by applying a binder thereto, which may be a heat curable foamed binder, such as a foamed acrylic binder. The binder is applied on top of the wads and is drawn into the wads and into the carrier by applying suction from beneath the carrier. The wads and the carrier are then heated to dry and chemically cross-link the binder. Accordingly, the apparatus has a suitable binder applying means and heating means.

Alternatively, the finely divided material includes thermoplastic particles and the wad is then stabilized and bonded to the carrier merely by heating the wad and the carrier, the apparatus only having a heating means.

The method may include moulding the wads and associated particles of the carrier to have a desired configuration, before parting, by a suitable moulding means. The wads may be moulded prior to stabilization and bonding or during stabilization and bonding. Further the wads may be moulded after application of the binder thereto, but before curing thereof.

As indicated in the introduction, a liquid impervious backing is normally required and such a backing may be laminated to the wad and the carrier so that the wad is sandwiched between the carrier and the backing. The apparatus may thus include an appropriate laminating means.

The finely divided material may be fibrous. A single type of fibre may be used or different fibres may be used. A cellulosic material such as wood pulp, cotton, rayon or the like may be used. Instead, or in addition, non-cellulosic materials such as polyolefinic or polyester fibres may be used.

Pressure applied during laminating or moulding causes the formation of a densified layer on the wad, similar to a layer of tissue, which serves to maintain the moulded shape and integrity of the wad.

It will be appreciated that the wads may have any suitable shape. Thus they may have regular shapes, eg. circular, oval, rounded rectangular, or trapezoid, or non-regular shapes, eg. c-shape, dumbell shape, bow-tie-like or butterfly-like.

With the present invention, problems normally associated with such composite products which are formed by cutting an absorbent layer into wads of a desired shape after the layer has been formed, such as wastage of finely divided material as a result of the offcuts of the absorbent layer; edges in which the finely divided materials in the wads are exposed or open, ie not sealed; hard and uncomfortable products; dusting-out of the finely divided materials in use; and delamination due to movement of a user's body against it, are avoided or at least reduced. Still further, by appropriate selection of the binder, the properties of the wad can be varied as desired. For example the fluid transfer capability of the wad, and the fluid transfer rate through the wad, can be selected as desired. In addition, when the wads are shaped as hereinbefore described, there is the further advantage of the wad shape being conformed to the shape of the body part with which it is to be used, eg the breast, ensuring greater comfort and efficacy.

The invention is illustrated in non-limiting manner by reference to the accompanying drawings in which:-
FIGURE 1 is a vertical view of one embodiment of an apparatus according to the invention;
FIGURE 2 is a plan view of the apparatus of Figure 1;
FIGURE 3 is a view of a former in the shape of a ring for use in the apparatus;
FIGURE 4 is a view of a second embodiment of the invention taken from a position which would be along line IV of Figure 1;
FIGURE 5 is a view of one embodiment of a wad forming station having a ring former;
FIGURE 6 is a view of a third embodiment of an apparatus according to the invention, looking from the end of the apparatus to the ring;
FIGURE 7 is a side view of the apparatus of Figure 1;
FIGURES 8a, 9a, 10a and 11a schematically show different arrangements for the entry of the supply fluid with fibres suspended therein;
FIGURES 8b, 9b, 10b and 11b illustrate the shape of deposits of fibres obtained in the apertures, following the arrangements of Figures 8a through 11a respectively;
FIGURE 12 shows one preferred form of contoured wad that is obtained using the arrangement of Figure 11a;
FIGURE 13 illustrates a second form of contoured wad that can be produced with the invention;
FIGURE 14 is a cross-section of a multilayered wad formed according to the invention;
FIGURE 15 is a vertical section through an apparatus according to the invention for making the wad of Figure 14;
FIGURE 16 shows, diagrammatically, a side view of apparatus according to another embodiment of the invention;
FIGURES 17, 18 and 19 show, in part, sectional views of portions of the apparatus of Figure 16, in use;
FIGURES 20, 21 and 22 show similar views of Figures 17, 18 and 19 respectively, of an apparatus similar to that of Figure 16, while FIGURE 23 shows diagrammatically the composite product formed with the apparatus of Figures 20, 21 and 22, in use;
FIGURE 24 shows a side view of apparatus according to another embodiment of the invention;
FIGURE 25 shows a side view of apparatus according to a further embodiment of the invention;
FIGURE 26 shows in greater detail a portion of a forming screen of the apparatus of Figures 24 and 25;
FIGURE 27 shows a perspective view of a wad formed with a forming screen as shown in Figure 26; and
FIGURES 28 and 29 show plan views of portions of carrier strips carrying a number of wads as produced by the apparatus of Figure 24 or Figure 25.

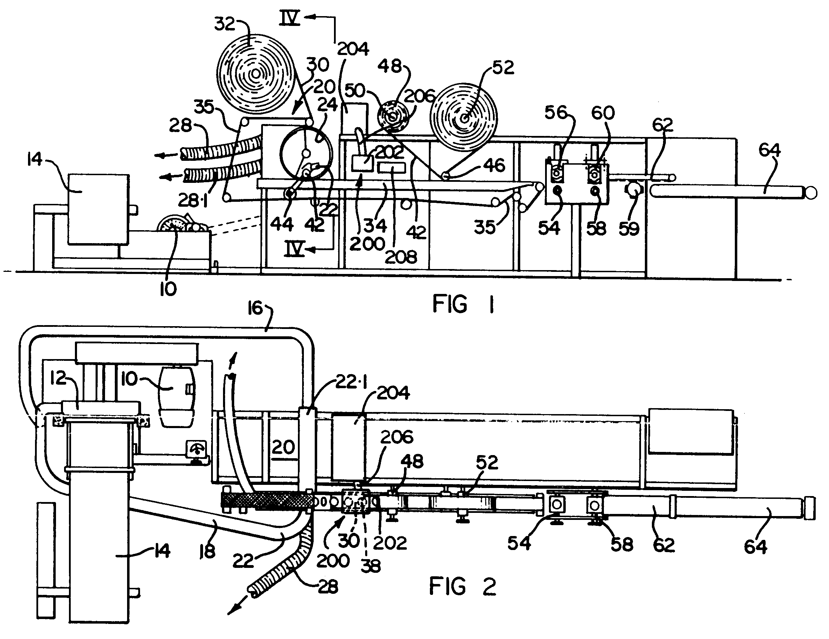

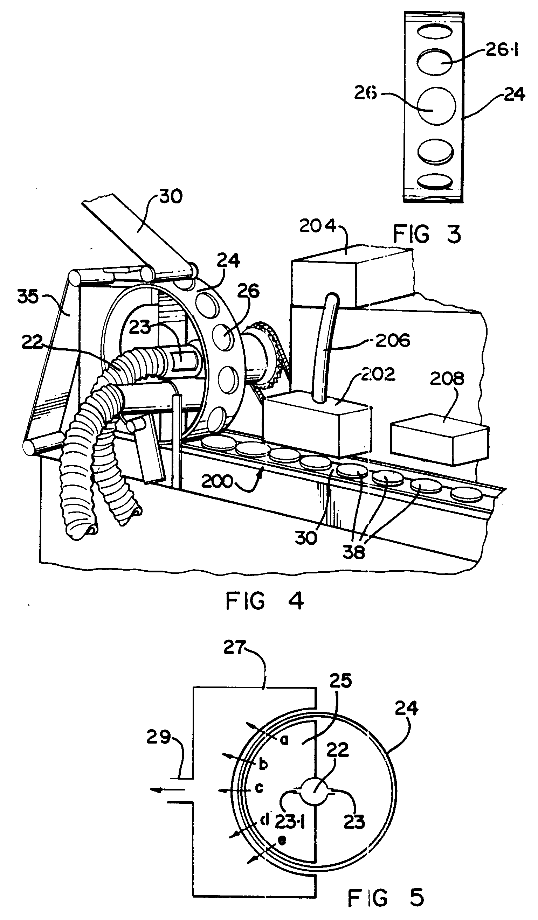

In Figures 1 to 5, a motor shown generally at 10 drives a mill 12. Raw pulp is supplied to the mill from a feed 14. The pulp is any suitable pulp, eg wood or other fibres. The finely-divided pulp so formed passes along supply pipes 16, 18 together with air as the conveying fluid to enter a wad-forming station shown generally at 20 in the shape of a forming box. The pipes enter the station transversely and from opposing sides at 22, 22.1 into the interior of a ring 24 as shown in more detail in Figure 5. In Figure 1, both pipes 22, 22.1 carry fibres suspended in air. In Figure 4 only one large transverse pipe 22 is provided to carry air with fibres suspended in it, while a smaller transverse pipe (not visible) permits air to enter from the opposing side.

A plan view of the ring 24 is illustrated in Figure 3 and, as can be seen, it contains a plurality of apertures 26, 26.1 etc passing through the circumference of the ring. The apertures are circular for forming breast pads but rings with other shaped apertures can be provided. The station 20 has the fibres entering from both the left and right sides at 22 and 22.1. An air inlet port shown at 23 (see Figure 4) is provided immediately in front of the pipes 16, 18. Air outlet pipes 28, 28.1 are provided at the back of the station 20. A vacuum is applied through the pipes 28, 28.1 to enable a pressure difference to arise between the inside of the ring where the finely divided pulp is supplied through the pipes 16, 18, and the outside of the ring which is covered by a continuous air-permeable liquid-pervious strip 30 of non-woven fabric from supply reel 32. This continuous strip, which forms the carrier, passes around the outside of the ring 24 on a continuous fluid permeable supporting belt 35. The strip 30 is then taken away from the ring 24 on the supporting belt 35. Vacuum is applied below the supporting belt 35 at vacuum chamber 34 to maintain the formed wads in position on the air permeable strip 30.

In the station 20, the strip 30 of air-permeable non-woven material closes off one side of the apertures 26, 26.1 etc whereby the finely divided material moves into the apertures and form a predetermined shape of wad on the strip, which is now the carrier 30. By providing circular apertures in the ring, a circular wad 38 will be formed on the carrier 30 as it passes through the station 20.

Provided in the interior of the ring 24 is a brush 42 driven by a pulley drive 44. Alternatively the brush can be positioned outside the ring. The brush 42 brushes off loose fibres which project above the required size of the wad to be formed. The thickness and shape of the wad may be adjusted by adjusting the flow of the pulp from one side. The effect on the thickness of the wad by reducing flow from one side causes a reduction on the other side. If two fibre streams having different compositions are employed blending of the fibres is achieved. Thus, superabsorbent fibres may be mixed in the wad with normal long fibre wood pulp or short fibre wood pulps may be mixed with staple length thermoplastic fibres. Separate mills and inlet pipes may lead to the wad-forming station to provide such wads.

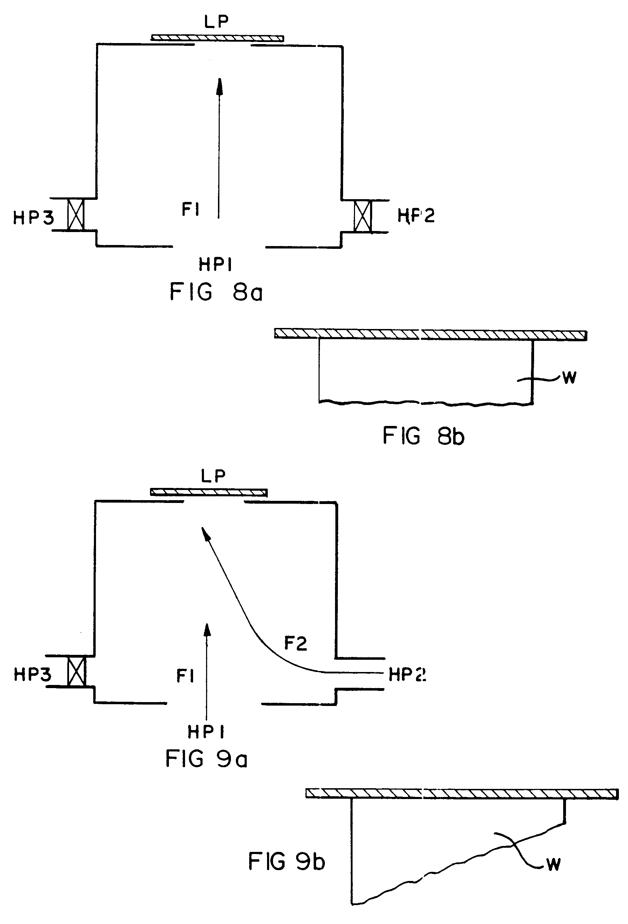

By adjusting the supply so that the amount, or size,or nature of the pulp is not the same from both fluid inlets, contoured wads can be obtained. This is explained more fully below by reference to Figures 8 through 11. If wads of contoured thickness are to be formed, the height of a brush 42 can also have a contour to act at more than one level. Such wads are more fitting to the contours of the part of the human anatomy to receive such wads. This can make the absorbent pads formed from the wads more comfortable and effective for absorbing body fluids without leakage around the sides of pads.

The wads 38 on the carrier 30 pass along and above the vacuum chamber 34, to a foamed binder application station generally indicated by reference numeral 200. In the station 200, vacuum is still applied to the underside of the carrier 30, while heat-curable foamed acrylic binder is applied, by means of an applicator 202 connected by means of a conduit 206 to a bulk vessel 204, to the top of the wads 38. The binder is an acrylic polymer emulsion supplied by Supacryl (Pty) Ltd under the Registered Trade Mark PRIMAL HA8 to which has been added a surfactant manufactured by Rohm & Haas Inc and sold under the name TRITON GR5M (also a Registered Trade Mark) to assist in the formation of a stable foam. The binder is drawn into the wads 38 and the carrier 30 as a result of the vacuum. The wads 38 then pass to a heat application station in which heat from a heater 208 dries and chemically cross-links the binder, thereby binding the material in the wads together as well as to the carrier 30, so that stabilized wads 38 are formed. The wads 38 are initially heated to 100 - 120°C and then heated further to about 160°C. The wads may, instead, be a mixture of wood pulp board and polyethylene fibres such as PULPEX manufactured by Hercules Inc. in a ratio of about 3:1. Such wads which contain thermoplastic fibres may by-pass the station 200, and may merely be stabilized by the heater 208, ie by application of heat thereto, at about 100°C to 120°C.

The stabilized wads 38 pass to a position 46 where a liquid-impermeable layer 48 of Surlyn or other material is supplied from a reel 50. In addition, an additional backing layer is supplied from reel 52. The backing layer may be slip-resistant and/or have adhesive applied to it.

The sandwich comprising the fluid-pervious carrier 30 with the stabilized shaped wads 38 on it and covered successively by a Surlyn layer 48 and backing layer are supplied to a station 54 where a sealer and embosser 56 seals the layers together. Thereafter, they pass to a station 58 where a rotary knife 60 cuts the sealed and embossed final absorbent pads from the continuous layers of material. The continuous layers, now with holes therein, pass around roller 59 and are sent to waste while the absorbent pads pass along vacuum conveyer belt 62 onto a further conveyor belt 64 from whence they are sent for packaging.

In Figure 5, the ring is shown at 24, the pulp inlet at 22, and air inlet port at 23. Inside the ring 24 is an inner chamber 25 which is open at its circumferential side. An outer chamber 27 is of lower pressure and air, together with fibres pass from 22 through opening 23.1 into inner chamber 25 and then through the apertures in the ring. The air then enters the outer chamber 27, and escapes through 29.

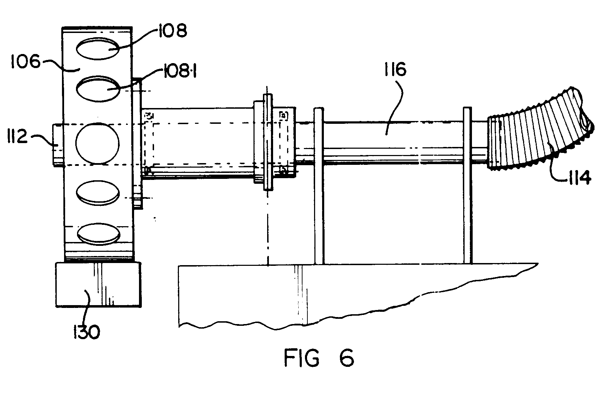

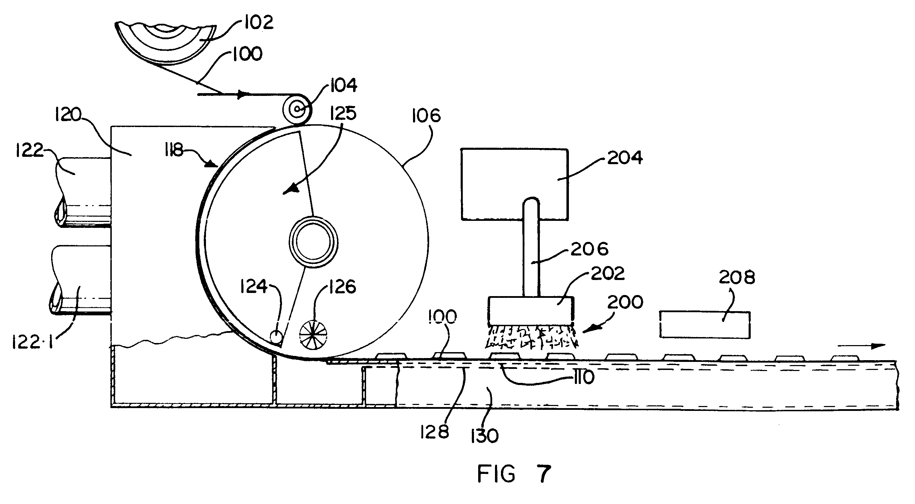

Referring now to Figures 6 and 7, a strip 100 of airpermeable material passes from a stock 102 over roller 104 and around a ring 106 having twelve wad-forming apertures 108, 108.1 etc in its circumference. The band 100 passes along a continuous air-pervious conveyor belt 110.

Air enters the interior of the ring 106 transversely of the movement of the band 100 from one side through axial inlet 112. Air with finely divided wood pulp suspended therein passes along flexible hose 114 into inlet pipe 116 and enters the ring axially from the other side. Thus the flow of air and pulp, and the flow of air into the ring is transversely of the direction of movement of the band 100.

A seal 118 is provided around the ring edge and a vacuum applied to vacuum box 120 through vacuum pipes 122 and 122.1. A free rotating roller 114 within inner chamber 125 reduces the leakage of air into the forming station at the most likely point of maximum leakage.

A brush 126 is provided for removing excess finely divided material. The continuous conveyor belt 110 runs on a perforated upper plate 128 of vacuum box 130 to assist in removing pulp from the aperture pockets of the ring by applying a vacuum to box 130.

An appropriate electrical panel, drive means and other suitable accessories can be provided.

Figures 8 through 11 will now be described as they illustrate the effect of varying the fluid flow.

In Figure 8a, there exists a fluid flow (F1) from a high pressure inlet (HP1) to low pressure outlet aperture (LP), ie aperture 26 of the ring 24 of Figure 3. HP2 and HP3 are closed and are the inlets 22 and 22.1 of Figure 3. A wad (W) of the shape shown in figure 8b is obtained if finely divided materials are introduced into fluid flow F1.

In Figure 9a with high pressure inlet (HP2) now open fluid flow F2 is caused to change direction by fluid flow F1 towards aperture LP. In this situation it is found that finely divided materials entering at HP2 are deposited in the aperture LP as shown in cross section in Figure 9b.

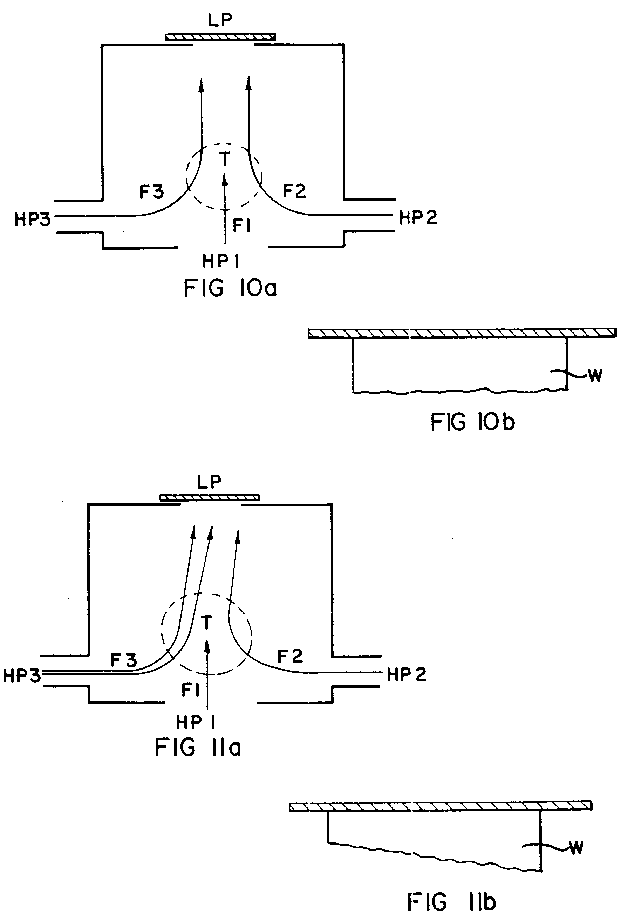

In Figure 10a with high pressure inlet (HP3) also open, fluid flow F3 is also caused to change direction by F1 towards LP. If F2 and F3 are open by approximately equal amounts the resultant fluid flow is almost normal to the plane of LP and even thicknesses of finely divided materials are deposited as shown in cross section in Figure 10b. Because of the turbulence at T it is not important (for single component wads) whether the finely divided materials are supplied through HP2 or through both HP2 and HP3. If the wad comprises more than one component then separate finely divided materials can be supplied through separate high pressure inlets. Mixing of the two components can occur in the space where turbulence occurs.

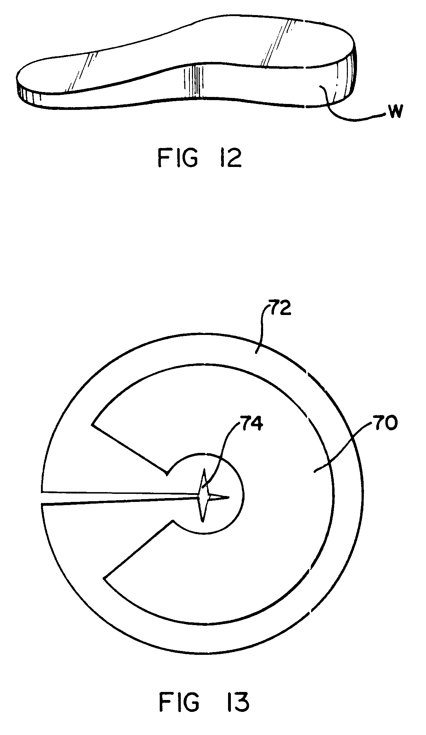

In Figure 11a, F3 is open by a greater amount than F2 and the resultant fluid flow to LP is displaced from normal by the unequal flows. A wad of cross section 11b illustrates the deposition of the finely divided materials. This contoured deposition is particularly useful for anatomically conforming sanitary pads.

Figure 12 shows a typical shape for wad W obtained according to Figure 11a.

The invention enables one to manufacture very stable absorbent pads because the wad defining the central absorbent layer is formed directly on a carrier and is attached thereto. The wad is then stabilized by foam-bonding or heat bonding (in the case of wads containing thermoplastic particles), and is thereby also bonded to the carrier. When the wad contains powders such as superabsorbents, the bonding inhibits shaking out thereof through the outer layers during storage, transportation or in use.

With the invention, one is able to form more intricate shapes. For example, one can make 'doughnut shaped' surgical dressings for application to tracheostomy drains and the like by cutting a circular aperture with a segment retained. The incomplete disc so formed can be sealed between two layers. By subsequent cutting it can form an effective shaped dressing pad. Such intricate designs were not able to be achieved with the prior art processes known to us because the friable wad became broken or disturbed during transfer from the forming means onto the carrying belt. With the present invention, intricate pads can be formed in their final position on one of the outer layers of the product.

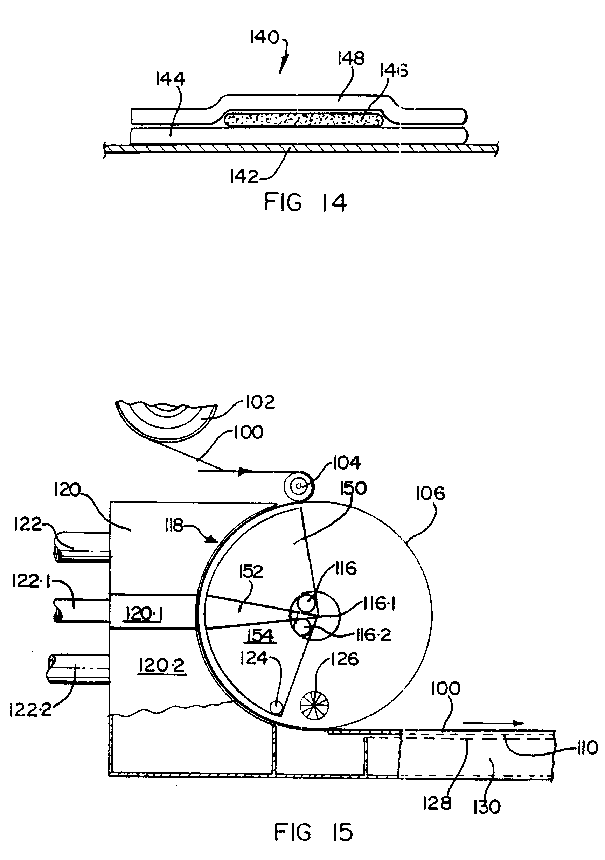

A surgical dressing of 'doughnut shape' is illustrated in Figure 13. This dressing comprises an absorbent wad 70 of the segmented doughnut shape illustrated. The wad 70 is formed using an aperture of generally C-shape. The wad 70 is applied to a carrier 72 and a centre cut 74 made for fitting around a wound drain. The wad 70 is formed directly on the carrier 72, so no transfer step takes place. Although Figure 13 illustrates a substantially round product it is understood that similar products with wads having substantially straight sides are also possible with the invention.

Referring now to Figure 14, pad 140 comprises a fluidpermeable layer 142 on which has been deposited a first layer 144 of one type of finely divided material (f.d.m.1). A second layer 146 of a different finely divided material (f.d.m.2) is deposited on the first layer 144, and a third layer 148 of a still different finely divided material (f.d.m.3) is deposited on the other layers.

In Figure 15, the apparatus is similar to that of Figure 7 and similar numbering is used. A strip 100 of air-permeable material passes from a stock 102 over roller 104 and around a ring 106 containing twelve wad-forming apertures. The strip 100 passes along a continuous air-pervious conveyor belt 110.

The interior of the ring 106 is divided into three forming stations 150, 152 and 154 which are separate chambers each with a separate pair of facing fluid inlets of which one inlet 116, 116.1 and 116.2 per chamber is visible. The stations 150, 152 and 154 are at a higher pressure than downstream vacuum boxes 120, 120.1 and 120.2. Vacuum pipes 122, 122.1 and 122.2 lead from each of the vacuum boxes 120, 120.1 and 120.2.

A free rotating roller is shown at 124 and a brush at 126. The strip 100 runs on continuous conveyor belt 110, which passes over perforated upper plate 128 of vacuum box 130 in a similar manner to that illustrated in Figure 7.

The separate chambers forming the stations 150, 152 and 154 are open at their circumferential sides to permit the finely divided material passing through them to be deposited in the apertures. The central station 152 is only partially open at its circumferential side, thereby restricting deposition of the finely divided material in the central portion of the apertures.

Instead of having a single mill, a plurality of mills, eg two or more, can be utilized. Similarly a plurality of forming stations can be used, eg to provide multilayered wads.

The apertures in the former may have vertical or contoured sides as desired.

In an embossing station, appropriate embossing to provide wicking can be incorporated. Any suitable wicking, eg wicking to move fluid rapidly from the centre of the pad towards the outside can be embossed on the pads.

It is to be understood that the carrier generally forms the body-facing layer in use. Other materials suitable for use as the carrier include fluid-impervious vapour-permeable materials such as spunbonded non-woven fabrics. The carrier then becomes a backing layer of the finished pad.

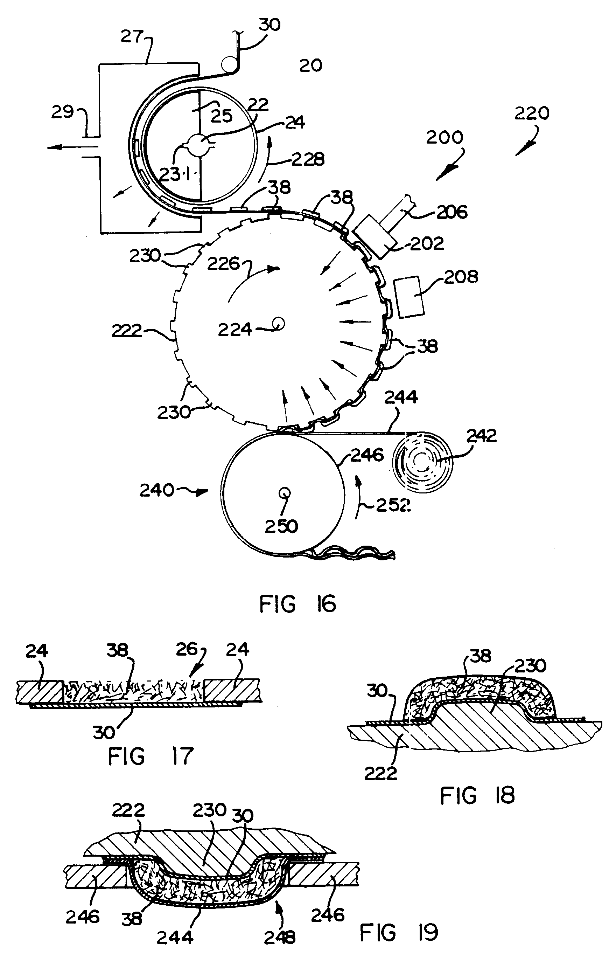

Referring to Figures 16 to 19, reference numeral 220 generally indicates apparatus according to another embodiment of the invention.

Parts of the apparatus 220 which are the same or similar to parts of any of the apparatus hereinbefore described, are indicated with the same reference numerals.

The apparatus 220 includes a forming ring 24, and its associated components, as hereinbefore described with particular reference to Figures 1 to 5.

Below the ring 24 is located a foraminous ring 222, mounted to rotate about an axis 224 extending parallel to the axis of the ring 24. The ring 222 is driven to rotate by means of suitable drive means (not shown) to rotate in a clockwise direction as indicated by arrow 226, while the ring 24 rotates in an anti-clockwise direction, as indicated by arrow 228. The ring 222 has a plurality of circumferentially spaced foraminous dome- or dish-shaped moulds 230 protruding therefrom. The ring 222 is also provided with vacuum generating means (not shown) for creating a fluid pressure difference between the inside and the outside of the ring, with the inside of the ring being at the lower fluid pressure. The binder applicator 202 and heater 208 are located adjacent the ring 222, with the vacuum required during the binder application and heat curing, as hereinbefore described, thus naturally being supplied by the vacuum drawn on the inside of the ring 222.

The apparatus 220 also includes a backing application station, generally indicated by reference numeral 240. The backing application station 240 comprises a roll or reel 242 containing a continuous length of liquid-impermeable Surlyn material 244, with the material 244 passing around a heated apertured ring 246. The apertured ring 246 is provided with a plurality of circumferentially spaced apertures 248, similar to the apertures 26 in the ring 24. The ring 246 is mounted to rotate about an axis 250 which extends parallel to the axis 224 of the ring 222, and it is driven to rotate by means of suitable drive means (not shown) in an anti-clockwise direction as indicated by arrow 252. Thus, as it rotates, its apertures 248 move into register with the protruding moulds 230 on the ring 240 so that the moulds 230 can protrude successively into openings 248 in the ring 246. The backing material 244 is fed into the nip of the rings 222, 246.

In use, as the carrier 30 with the wads 38 located thereon exits the forming station 20, each wad 38 is located over one of the dome-shaped protruding moulds 230 on the ring 222 with the carrier 30 being in contact with the moulds 230. On application of the binder by means of a binder applicator 202 and heat by means of the heater 208, while maintaining the inside of the ring 22 under vacuum, the wads 38 are conformed to the shape of the moulds 230, and thus retain this shape once the binder has cured.

In the station 240, the material 244 is applied as a backing layer over the shaped stabilized wad 38 while the wad 38 is still located over the mould 230. The ring or drum 246 is heated so that, as the backing layer 244 is applied, it is immediately sealed to the carrier 30, as indicated most clearly in Figure 19.

With the apparatus 220, a moulded contoured breast pad can thus be formed.

In another embodiment of the apparatus 220 (not shown), the binder application station 200 can be located between the station 20 and the ring 222, immediately above the wads 38 located on the carrier 30, with suitable vacuum generating means for removing excess foamed binder being provided below the carrier 30 and the station 200. The ring 222 can then be in the form of a relatively simple non-foraminous or non-apertured heated drum with raised moulds on its outer surface and without any vacuum generating means on its inside.

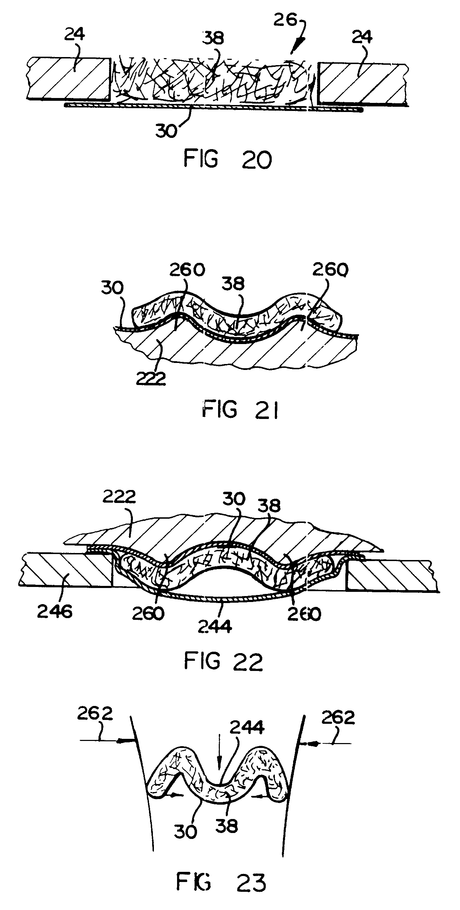

Referring to Figures 20 to 23, apparatus substantially the same as the apparatus 220 hereinbefore described with reference to Figures 16 to 19, is shown in part. However, the protruding moulds 230 are somewhat differently shaped and instead are in the form of pairs of protruding ridges 260 spaced apart across the ring and extending around discrete arcs of the ring 222. The moulded contoured wad 30 thus assumes a shape as indicated in Figures 21 and 22, and in this manner a moulded shaped sanitary pad is formed.

This pad has the advantage that, in use, under thigh pressure as indicated by arrows 262 in Figure 23, it conforms closer to the shape of the vagina.

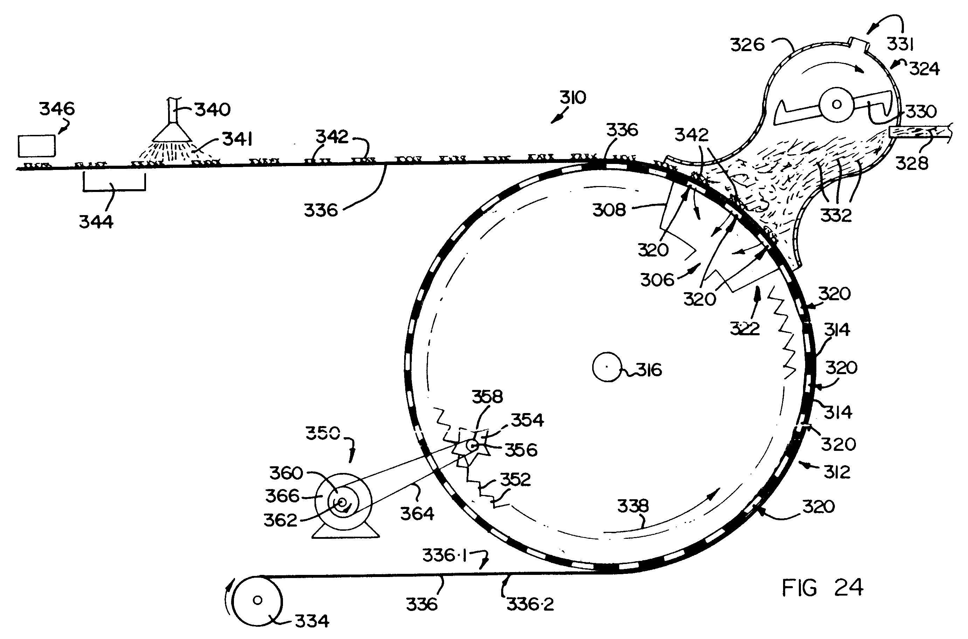

Referring to Figure 24, reference numeral 310 generally indicates apparatus according to a further embodiment of the invention.

The apparatus 310 includes a circular cylindrical drum, generally indicated by reference numeral 312. The drum 312 has a cylindrical wall 314. The drum 311 is adapted to be driven about a rotational axis 316 by means of suitable drive means generally indicated by reference numeral 350.

The drive means 350 comprises a plurality of radially inwardly protruding circumferentially spaced teeth 352 on the drum 312. Two sets of teeth 352, located respectively at the ends of the drum 312, are provided. The drum 312 is rotatably supported by two side plates (not shown) which have raised circular portions (also not shown) on their inner surfaces. The internal diameter of these portions is slightly larger than the outer diameter of the drum and are lined with a suitable non-slip material or with bearings. With each set of teeth 352 is associated a toothed cog 354, with the cogs 354 being mounted on a single shaft or axle 356. To one end of the shaft 356 is mounted a pulley 358 which is aligned with a pulley 360 mounted to the output shaft 362 of an electric motor 366. An endless belt 364 connects the pulleys 358, 360.

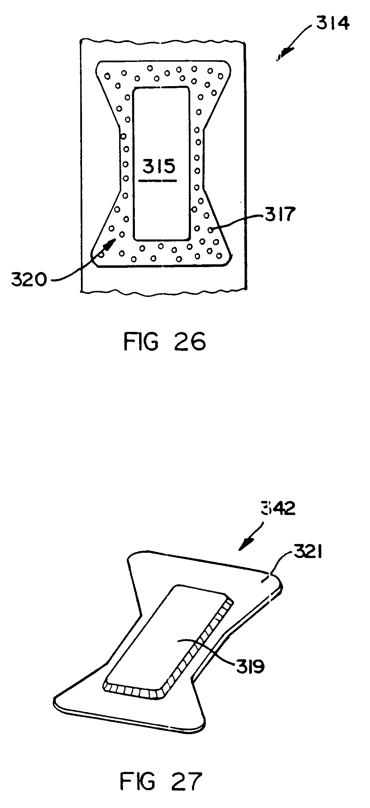

The cylindrical wall 314 of the drum 312 is generally imperforate, (ie. solid) with forming zones 320 in the form of apertures of predetermined shape, being provided therein. The apertures 320 are described in more detail below with reference to Figure 26.

The apparatus 310 also includes a vacuum generating means for applying a vacuum to the inside of the drum wall 314, at a forming station 322. The vacuum generating means includes a vacuum box 308 with a duct 306 communicating with a vacuum source (not shown). The box 308 is located within the drum 312, in contact with its wall 314. A pressure differential is formed across the drum wall with a higher pressure being present on the outside and a lower pressure on the inside.

The apparatus 310 also includes a supply means, generally indicated by reference numeral 324, for supplying shredded defiberized pulp fibres to the outside of the drum wall 314, at the station 322. The supply means 324 comprises a housing 326, a pulp sheet inlet 328 leading into the housing 326, a rotatable shredder 330 located within the housing, drive means (not shown) for driving the shredder 330 to rotate it, and an air inlet 331 so that air can enter the housing. Thus, the required air flow inside the housing is generated by the movement of the shredder and the vacuum generating means. As the shredder 330 rotates, it shreds pulp sheet entering the inside of the housing via the inlet 328 into defiberized pulp fibres 332 which are entrained in the air.

The apparatus 310 still further includes a driven bobbin or roll 334 of non-woven fabric located such that a strip 336 of the non-woven fabric can be fed therefrom directly onto the outside of the drum wall 314, ahead of the supply means 324, as the drum 312 rotates in the direction indicated by arrow 338.

The apparatus 310 still further includes drawing off means (not shown) for drawing off the strip 336 from the drum wall 314 on the other side of the supply means 324. The drawing off means typically comprises a driven wind-up unit, and driven rollers for supporting the strip 336, as required.

The apparatus 310 also includes a spray nozzle 340 for applying a foamed acrylic binder to wads 342 of shredded fibrous pulp formed on the strip 336. A vacuum-generating means 344, generates a vacuum below the strip 336 downstream of the nozzle 340 to spread the foamed binder through the wad and onto the strip 336, Heating means, generally indicated by reference numeral 346, is located above the strip 336, downstream of the vacuum generating means 344 for drying and chemically cross-linking the binder to stabilize the wads 342 and bond them to the strip 336. Typically, the heating means 346 can be steam heated cans. When the wads 342 contain thermoplastic particles, the nozzle 340 and vacuum generating means 340 can be dispensed with, with the wads 342 then being stabilized and bonded directly by passing through or over the heating means 346.

In use, as the drum 312 rotates in the direction of arrow 338, a continuous strip 336 of non-woven fabric is unwound directly from the bobbin 334 so that its surface 336.1 abuts against the outside surface of the drum wall 314. At the station 322, as a result of the vacuum generated by the box 308 and the apertures 320, air passes through the strip 336 only in those areas in alignment with the apertures 320. As the fibres 332 are entrained in the air, the fibres 332 are deposited on surface 336.2 of the strip 336 in the said areas to form the wads 342, while no fibres are deposited elsewhere on that portion of the strip 336 in the forming station 322. The strip 336 with the wads 342 located thereon then pass to the nozzle 340 where the wads 342 are stabilized as hereinbefore described.

After composite products comprising the stabilized wads 342 attached to the strip 336, are formed, they can be laminated to a plastics film backing, eg a polythene film backing, whereafter the polythene film backing and the non-woven fabric can be cut to the same shape (but larger) as the wads. In this fashion soft panty-liners, and the like can be formed. Instead they can be laminated onto an absorbent layer, eg a peatmoss layer, which can then in turn be laminated onto a polythene film backing layer, to form sanitary pads or the like.

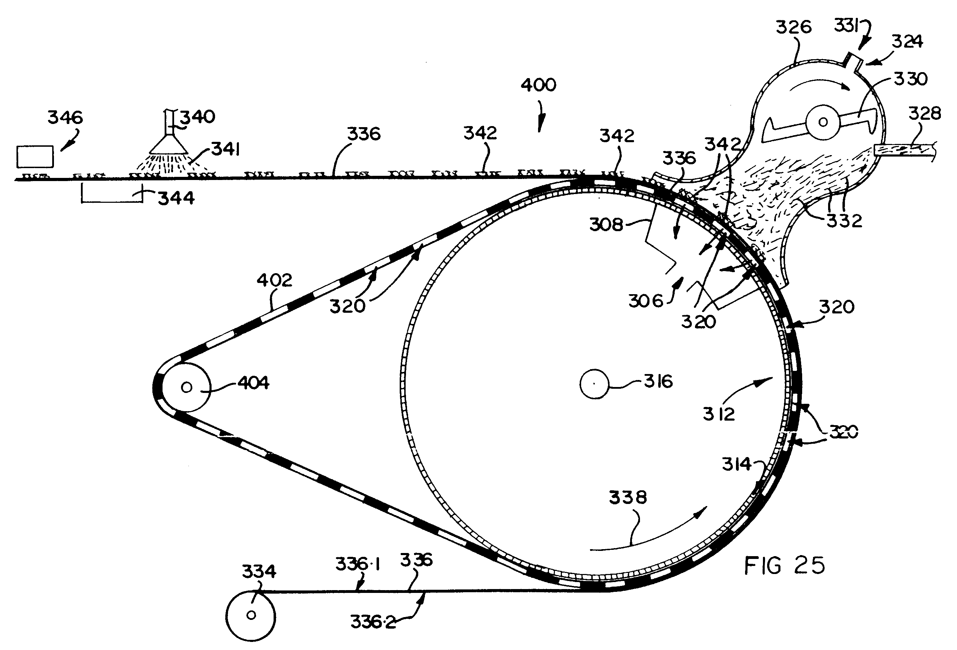

Referring to Figure 25, reference numeral 400 generally indicates apparatus according to a further embodiment of the invention.

Parts of the apparatus 400 which are the same or similar to the apparatus 310, are indicated with the same reference numerals.

The apparatus 400 also has a drum 312 having a sieve-like, perforated circular cylindrical wall 314. The drum 312 of the apparatus 400 can be driven by the same drive means as the drum 312 of the apparatus 310. However, the apertures 320 are not provided in the drum wall itself. Instead, the apparatus 400 includes a continuous imperforate belt 402 which passes around the drum 312 as well as around a pulley, idler or roller 404. The position of the idler 404 can be adjusted, thereby to vary the tension in the belt 402. The apertures 320 are provided in the belt 402. Thus, as the drum 312 rotates, the belt 402 continually engages a portion of the drum wall. After the strip 336, with the wads 342 deposited thereon, has been drawn off, the belt disengages from the drum wall 314, and passes around the pulley 404.

The Applicant believes that use of the belt 402 can have advantages. For example, the desired shape of the wads 342 can be varied easily, by merely selecting a belt 402 having apertures 320 of different shapes or sizes. Furthermore, the apparatus is not restricted to sizes and shapes of apertures 320 being selected such that they fit exactly into the drum wall 314. The Applicant further believes that drum blockages on extended runs can be avoided or reduced with the apparatus 400.

The apertures 320 can be of any desired shape, depending on the end use. For example, they may be dogbone-shaped or butterfly-shaped if it is desired to provide panty-liners or sanitary pads respectively.

Referring to Figure 26, a portion of the wall 314 (or the belt 402) is shown. The aperture 320 has a relatively large, rectangular opening 315 with a surrounding "bow-tie" portion 317 with holes 319 therein. The opening 315 provides a region with unrestricted permeability and the portion 317 with its holes 319 provides a region with intermediate permeability.

A wad 342 that is produced with an aperture 320 as shown in Figure 26, is shown in Figure 27. Thus, the wad 342 has a substantially "bow-tie" external profile with a central rectangular portion 319 and a border 321. The central portion 319 is thicker than the border 321, as more air passes through that area of the strip 336 in alignment with the opening 315 than that in alignment with the portion 317, and therefore more fibres are collected in the middle than on the sides.

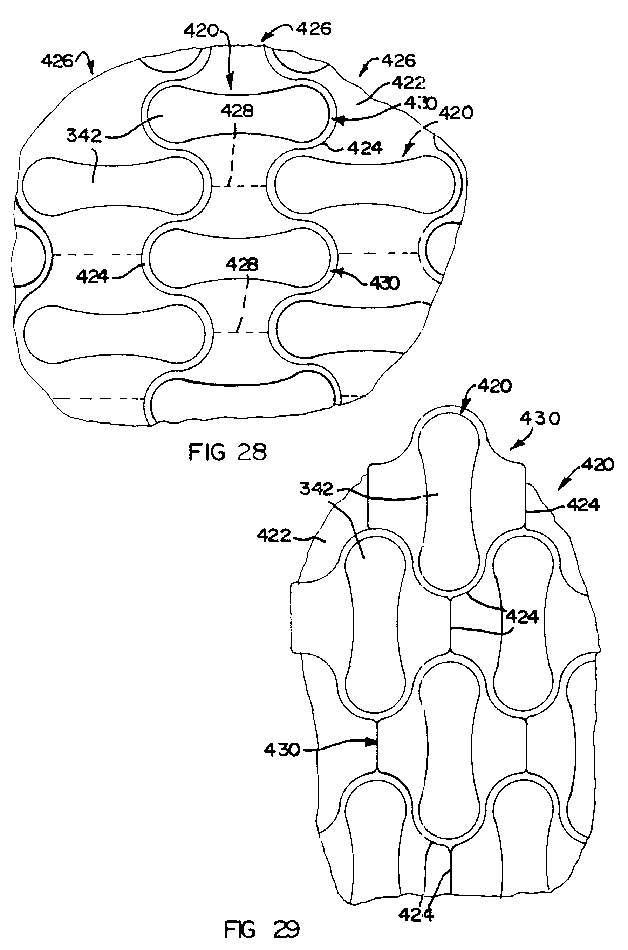

It will be appreciated that with the apparatus 310, 400, the drum 312 can be made of any desired length, so that a number of wads can be formed side-by-side simultaneously. The apertures 320 need then naturally not be aligned with each other along the length of the drum wall 314, but can be staggered so that rows 420 (see Figures 28 and 29) of composite products, each comprising a layer of non-woven fabric and wads 342 of stabilized defiberized pulp particles located thereon as described, and a layer 422 of fluid-impervious material, eg a plastics film backing, laminated thereto, are produced. In a cutting station (not shown) these rows can then be separated from one another by cutting along more-or-less sinusoidal, generally longitudinally extending, cut lines 424, into strips 426 of composite product. The strips 426 can then be cut along transversely extending cut lines 428 to produce individual composite products 430 comprising pads with side flaps. In the case of the arrangement as shown in Figure 29, the individual composite products 430 are formed once the cuts along the cut lines 424 have been effected, so that the additional cuts along transverse cut lines are not required.

## Claims

1. A method of manufacturing a composite product, comprising the step of forming a discrete wad (38, 70, 342) of finely divided material on a fluid permeable carrier (30, 100, 142, 336) by generating a pressure differential across the carrier; defining an area on the carrier through which a transporting fluid may pass, as a result of the pressure differential, by means of a former (24, 106, 314, 402); and introducing the finely divided material into the fluid such that the finely divided material is deposited on the carrier in the area defined by the former, to provide a border around the wad; said method being characterised in that it also includes the steps of stabilising the wad by bonding at least some of the material thereof together; and bonding at least some of the material in the wad to the carrier by
(a) including thermoplastic particles in the finely divided material and heating the wad and the carrier, or
(b) applying a foamed binder on top of the wad and drawing the foamed binder into the wad and the carrier by generating a pressure differential across the wad and the carrier.

2. The method claimed in claim 1, characterised in that it includes parting that portion of the carrier to which the wad is bonded from the rest of the carrier.

3. The method claimed in claim 1, characterised in that the carrier is sheet-like, a plurality of discrete wads are formed thereon which are stabilised and bonded thereto and the carrier is parted to separate each wad and its associated portion of the carrier from the others.

4. The method claimed in any preceding claim, characterised in that there is a high pressure on one side of the carrier and a low pressure on the other side, the finely divided material being introduced on the high pressure side, and the former (24, 106, 314) is located on the high pressure side of the carrier.

5. The method claimed in any of claims 1 to 3, characterised in that there is a high pressure on one side of the carrier and a low pressure on the other side, the finely divided material being introduced on the high pressure side, and the former (312, 402) is located on the low pressure side of the carrier.

6. The method claimed in any preceding claim, characterised in that the carrier is supported by a fluid permeable support member (35) when the wad is formed thereon and when the wad is stabilised and bonded thereto.

7. The method claimed in claim 3, characterised in that the carrier is a strip that is drawn from a roll (132, 102, 334) thereof and the wads are formed thereon and stabilised and bonded thereto in a continuous manner.

8. The method claimed in any preceding claim, characterised in that the wad is stabilised and bonded to the carrier by applying a binder thereto.

9. The method claimed in claim 8, characterised in that the wad and the carrier are heated.

10. The method claimed in claim 2, characterised in that it includes moulding the wad and its associated portion of the carrier to have a desired configuration before parting.

11. The method claimed in claim 10, characterised in that the wad and associated carrier portion are moulded prior to stabilisation and bonding.

12. The method claimed in claim 10, characterised in that the wad and associated carrier portion are moulded during stabilisation and bonding.

13. The method claimed in claim 12, characterised in that the wad is stabilised and bonded to the carrier by means of a heat curable binder and the wad and associated carrier portion are moulded after application of the binder thereto, but before curing thereof.

14. The method claimed in claim 1, characterised in that it includes laminating the wad and the carrier to a liquid impervious backing (42, 244) so that the wad is sandwiched between the carrier and the backing.

15. The method claimed in claim 1, characterised in that the finely divided material is fibrous.

16. The method claimed in claim 1, characterised in that the carrier is a non-woven fabric.

17. An apparatus (200, 310, 406) for manufacturing a composite product having a discrete wad (38, 70, 342) of finely divided material on a fluid permeable carrier (30, 100, 142, 336), said apparatus including a forming means (20, 322) for forming the wad on the carrier, said forming means including a pressure differential generating means (27, 28, 120, 308) for generating a pressure differential across the carrier; a former (24, 106, 314, 402) for defining the desired area on the carrier through which a transporting fluid may pass; and an introducing means (16, 18, 25, 125, 150, 152, 154, 324) located on a side of the carrier which is, in use, at a higher pressure, for introducing the finely divided material, in use, into a stream of the transporting fluid to be deposited on the carrier in the defined area, characterised in that said apparatus also includes a stabilising and bonding means (200, 340, 344, 346) for stabilising the wad by bonding at least some of the material thereof together and to the carrier, said stabilising and bonding means including
(a) means for introducing thermoplastic particles into the finely divided material, and means for heating (346) the wad and the carrier, or
(b) means (202, 340) for a applying a foamed binder on top of the wad, and means (34, 130, 344) for generating a pressure differential across the wad and the carrier to draw the binder into the wad and carrier.

18. The apparatus claimed in claim 17, in which the carrier is larger than the wad, characterised in that it includes a parting means for parting (58) that portion of the carrier to which the wad is bonded from the rest of the carrier.

19. The apparatus as claimed in claim 18, characterised in that it includes a carrier supply means (32, 103, 334) for supplying a sheet-like carrier to the forming means and in which the forming means forms, in use, a plurality of discrete wads on the carrier; the stabilising and bonding means stabilises all the wads and bonds them to the carrier; and the parting means parts the carrier to separate each wad and its associated portion of the carrier from the others.

20. The apparatus claimed in claim 18, characterised in that the parting means parts the carrier, in use, a predetermined distance from the edge of the wad to provide a border thereabout.

21. The apparatus claimed in any of claims 17 to 20, characterised in that the former (24, 106) and the introducing means (16, 18, 25, 125, 150, 152, 154) are on the same side of the carrier.

22. The apparatus claimed in any of claims 17 to 20, characterised in that the former (312, 402) and the introducing means (324) are on opposite sides of the carrier.

23. The apparatus claimed in any of claims 17 to 22, characterised in that it includes a fluid permeable support means (35) for supporting the carrier.

24. The apparatus claimed in claim 19, characterised in that the forming means and the stabilising and bonding means are continuously operable for forming the wads on a strip and stabilising them thereon and bonding them thereto in a continuous manner, the carrier supply means supplying, in use, the strip from a roll thereof.

25. The apparatus claimed in any of claims 17 to 24, characterised in that the stabilising and bonding means includes a heating means (208, 346) for curing a heat curable binder.

26. The apparatus claimed in any of claims 17 to 25, characterised in that it includes a moulding means (56, 222) for moulding the wad and its associated carrier portion into a desired configuration.

27. The apparatus claimed in any of claims 17 to 26, characterised in that it includes a laminating means (46, 240) for laminating the wad and the carrier to a liquid impervious backing (42, 244) such that the wad is sandwiched between the carrier and the backing.

28. The apparatus claimed in any of claims 17 to 27, characterised in that the finely divided material is fibrous.

29. The apparatus claimed in any of claims 17 to 28, characterised in that the carrier is a non-woven fabric.

## Patentansprüche

1. Verfahren zur Herstellung eines zusammengesetzten Erzeugnisses, umfassend den Schritt des Formens eines einzelnen Watteteils (38, 70, 342) aus feinverteiltem Material auf einem flüssigkeitsdurchlässigen Träger (30, 100, 142, 336) durch Erzeugen eines Druckdifferentials durch den Träger; Bilden eines Bereichs auf dem Träger, durch den ein transportierendes Fluid aufgrund des Druckdifferentials mittels eines Formers (24, 106, 314, 402) hindurchgehen kann; und Einführen des feinverteilten Materials in das Fluid, derart, daß das feinverteilte Material auf dem Träger in dem durch den Former definierten Bereich abgelegt wird, um eine Einfassung um das Watteteil herum zu bilden, wobei das Verfahren dadurch gekennzeichnet ist, daß es auch die Schritte des Stabilisierens des Watteteils durch Miteinanderverbinden von mindestens einem Teil des Materials desselben sowie durch Miteinanderverbinden von mindestens einem Teil des Materials in dem Watteteil mit dem Träger umfaßt durch
(a) das Einschließen themoplastischer Partikel in dem feinverteilten Material und Erhitzen des Watteteils und des Trägers, oder
(b) Aufbringen eines geschäumten Bindemittels auf die Oberseite des Watteteils und Einziehen des geschäumten Bindemittels in das Watteteil und den Träger durch Erzeugen eines Druckdifferentials durch das Watteteil und den Träger.

2. Verfahren nach Anspruch 1, dadurch gekennzeichnet, daß es umfaßt: die Unterteilung desjenigen Teils des Trägers, mit dem das Watteteil verbunden ist, gegenüber dem Rest des Trägers.

3. Verfahren nach Anspruch 1, dadurch gekennzeichnet, daß der Träger schichtähnlich ist, eine Mehrzahl von einzelnen Watteteilen darauf geformt wird, die stablilisiert und mit dem Träger verbunden werden, und der Träger unterteilt wird, um jedes Watteteil und den zugehörigen Teil des Trägers von den anderen zu trennen.

4. Verfahren nach irgendeinem vorhergehenden Anspruch, dadurch gekennzeichnet, daß ein Hochdruck auf einer Seite des Trägers und ein Niederdruck auf der anderen Seite vorgesehen ist, wobei das fernverteilte Material auf die Hochdruckseite aufgegeben wird und der Former (24, 106, 314) auf der Hochdruckseite des Trägers angeordnet ist.

5. Verfahren nach einem der Ansprüche 1 bis 3, dadurch gekennzeichnet, daß ein Hochdruck auf einer Seite des Trägers und ein Niederdruck auf der anderen Seite vorgesehen wird, wobei das feinverteilte Material auf die Hochdruckseite aufgegeben wird und der Former (312, 402) auf der Niederdruckseite des Trägers angeordnet wird.

6. Verfahren nach irgendeinem vorhergehenden Anspruch, dadurch gekennzeichet, daß der Träger durch ein flüssigkeitsdurchlässiges Tragorgan (35) abgestützt wird, wenn das Watteteil auf diesem geformt wird und wenn das Watteteil stabilisiert und damit verbunden wird.

7. Verfahren nach Anspruch 3, dadurch gekennzeichnet, daß der Träger ein Streifen ist, der von einer Rolle (132, 102, 334) derselben abgezogen wird und das Watteteil auf diesem geformt und stabilisiert und damit fortlaufend verbunden wird.

8. Verfahren nach irgendeinem vorhergehenden Anspruch, dadurch gekennzeichnet, daß das Watteteil stabilisiert und mit dem Träger durch Aufbringen eines Bindemittels auf diesen verbunden wird.

9. Verfahren nach Anspruch 8, dadurch gekennzeichnet, daß das Watteteil und der Träger erhitzt werden.

10. Verfahren nach Anspruch 2, dadurch gekennzeichnet, daß es das Formen des Watteteils und seines zugehörigen Teils des Trägers umfaßt, um eine gewünschte Konfiguration vor der Unterteilung zu erreichen.

11. Verfahren nach Anspruch 10, dadurch gekennzeichnet, daß das Watteteil und der zugehörige Trägerteil vor dem Stabilisieren und dem Verbinden geformt werden.

12. Verfahren nach Anspruch 10, dadurch gekennzeichnet, daß das Watteteil und der zugehörige Trägerteil während des Stabilisierens und Verbindens geformt werden.

13. Verfahren nach Anspruch 12, dadurch gekennzeichnet, daß das Watteteil stabilisiert und mit dem Träger mittels eines hitzehärtbaren Bindemittels verbunden wird und das Watteteil und der zugehörige Trägerteil nach dem Aufbringen des Bindemittels auf diesen, jedoch vor dem Aushärten desselben, geformt werden.

14. Verfahren nach Anspruch 1, dadurch gekennzeichnet, daß es das Laminieren des Watteteils und des Trägers auf einer flüssigkeitsundurchlässigen Unterlage (42, 244) umfaßt, so daß das Watteteil zwischen dem Träger und der Unterlage gesandwicht wird.

15. Verfahren nach Anspruch 1, dadurch gekennzeichnet, daß das feinverteilte Material faserhaltig ist.

16. Verfahren nach Anspruch 1, dadurch gekennzeichnet, daß der Träger ein nicht gewebter Stoff ist.

17. Vorrichtung (200, 310, 406) zum Herstellen eines zusammengesetzten Erzeugnisses, das ein einzelnes Watteteil (38, 70, 342) aus feinverteiltem Material auf einem fluiddurchlässigen Träger (30, 100, 142, 336) umfaßt, wobei die Vorrichtung umfaßt: eine Formvorrichtung (20, 322) zum Formen des Watteteils auf dem Träger, wobei die Formvorrichtung eine ein Druckdifferential erzeugende Vorrichtung (27, 28, 120, 308) zur Erzeugung eines Druckdifferentials durch den Träger hindurch umfaßt; einen Former (24, 106, 314, 402) zum Definieren des gewünschten Bereichs auf dem Träger, durch den ein transportierendes Fluid hindurchgehen kann; und eine Zuführvorrichtung (16, 18, 25, 125, 150, 152, 154, 324), die auf einer Seite des Trägers angeordnet ist, und die, im Betrieb bei einem höheren Druck, zum Einführen des feinverteilten Materials, während des Betriebes, in einen Strom des transportierenden Fluides zum Ablegen auf dem Träger in dem definierten Bereich vorgesehen ist, dadurch gekennzeichnet, daß die Vorrichtung auch eine Stabilisierungs- und Verbindungsvorrichtung (200, 340, 344, 346) zum Stabilisieren des Watteteils durch Miteinanderverbinden von mindestens einem Teil des Materials desselben und mit dem Träger umfaßt, wobei die Stabilisierungs- und Verbindungsvorrichtung umfaßt:
(a) eine Vorrichtung zum Einführen thermoplastischer Partikel in das feinverteilte Material, und eine Vorrichtung (346) zum Erhitzen des Watteteils und des Trägers, oder
(b) eine Vorrichtung (202, 340) zum Aufbringen eines geschäumten Bindemittels auf die Oberseite des Watteteils, und eine Vorrichtung (34, 130, 344) zum Erzeugen einer Druckdifferenz durch das Watteteil und den Träger, um das Bindemittel in das Watteteil und den Träger zu ziehen.

18. Vorrichtung nach Anspruch 17, bei der der Träger größer als das Watteteil ist, dadurch gekennzeichnet, daß sie eine Unterteilungsvorrichtung (58) zum Unterteilen desjenigen Abschnitts des Trägers umfaßt, mit dem das Watteteil gegenüber dem Rest des Trägers verbunden ist.

19. Vorrichtung nach Anspruch 18, dadurch gekennzeichnet, daß sie eine Trägerzuführvorrichtung (32, 103, 334) für die Zufuhr eines schichtähnlichen Trägers zu der Formvorrichtung unfaßt und bei der die Formvorrichtung, im Betrieb, eine Mehrzahl von diskreter Watte auf dem Träger formt; wobei die Stabilisierungs- und Verbindungsvorrichtung alle einzelnen Watteteile stabilisiert und sie mit dem Träger verbindet; und die Unterteilungsvorrichtung den Träger unterteilt, um jedes Watteteil und ihren zugehörigen Abschnitt des Trägers von den anderen zu trennen.

20. Vorrichtung nach Anspruch 18, dadurch gekennzeichnet, daß die Unterteilungsvorrichtung den Träger im Betrieb in einem vorbestimmten Abstand von dem Rand des Watteteils unterteilt, um um diese herum eine Einhüllung zu bilden.

21. Vorrichtung nach einem der Ansprüche 17 bis 20, dadurch gekennzeichnet, daß der Former (24, 106) und die zuführvorrichtung (16, 18, 25, 125, 150, 152, 154) auf derselben Seite des Trägers angeordnet sind.

22. Vorrichtung nach einem der Ansprüche 17 bis 20, dadurch gekennzeichnet, daß der Former (312, 402) und die Zuführvorrichtung (324) auf gegenüberliegenden Seiten des Trägers angeordnet sind.

23. Vorrichtung nach einem der Ansprüche 17 bis 22, dadurch gekennzeichnet, daß sie eine fluiddurchlässige Tragvorrichtung (35) zur Abstützung des Trägers umfaßt.

24. Vorrichtung nach Anspruch 19, dadurch gekennzeichnet, daß die Formvorrichtung und die Stabilisierungs- und Verbindungsvorrichtung zum Formen der Watteteile auf einem Streifen und zum Stabilisieren derselben auf diesem und zum Verbinden derselben mit diesem in einer kontinuierlicher Weise fortlaufend betätigbar sind, wobei die Trägerzuführvorrichtung im Betrieb den Streifen von einer Rolle derselben zuführt.

25. Vorrichtung nach einem der Ansprüche 17 bis 24, dadurch gekennzeichnet, daß die Stabilisierungs- und Verbindungsvorrichtung eine Heizvorrichtung (208, 346) zum Aushärten eines wärmehärtbaren Bindemittels umfaßt.

26. Vorrichtung nach einem der Ansprüche 17 bis 25, dadurch gekennzeichnet, daß es eine Formvorrichtung (56, 222) zum Formen des Watteteils und seines zugehörigen Trägerteils in eine gewünschte Konfiguration umfaßt.

27. Vorrichtung nach einem der Ansprüche 17 bis 26, dadurch gekennzeichnet, daß es eine Laminiervorrichtung (46, 240) zum Laminieren des Watteteils und des Trägers auf einer flüssigkeitsundurchlässigen Unterlage (42, 244) umfaßt, derart, daß das Watteteil zwischen dem Träger und der Unterlage gesandwicht wird.

28. Vorrichtung nach einem der Ansprüche 17 bis 27, dadurch gekennzeichnet, daß das feinverteilte Material faserhaltig ist.

29. Vorrichtung nach einem der Ansprüche 17 bis 28, dadurch gekennzeichnet, daß der Träger ein nicht gewebter Stoff ist.

## Revendications

1. Procédé de fabrication d'un produit composite, comprenant l'étape qui consiste à former un garnissage discret (38, 70, 342) de matériau finement divisé sur un substrat perméable aux fluides (30, 100, 142, 336) par la production d'une pression différentielle à travers le substrat ; à définir sur le substrat, au moyen d'un gabarit (24, 106, 314, 402), une zone à travers laquelle peut passer un fluide de transport, en conséquence de la pression différentielle ; et à introduire le matériau finement divisé dans le fluide, de sorte que le matériau finement divisé est déposé sur le substrat dans la zone définie par le gabarit, pour réaliser une bordure autour du garnissage ; ledit procédé étant caractérisé en ce qu'il comprend aussi l'étape qui consiste à stabiliser le garnissage en collant au moins une partie de son matériau ; et à coller au moins une partie du matériau du garnissage au substrat
(a) en introduisant des particules thermoplastiques dans le matériau finement divisé, et en chauffant le garnissage et le substrat, ou
(b) en appliquant un liant sous forme de mousse au-dessus du garnissage, et en aspirant le liant sous forme de mousse dans le garnissage et le substrat en produisant une pression différentielle au travers du garnissage et du substrat.

2. Procédé selon la revendication 1, caractérisé en ce qu'il comprend l'étape consistant à séparer du reste du substrat la partie du substrat à laquelle le garnissage est collé.

3. Procédé selon la revendication 1, caractérisé en ce que le substrat est de type feuille sur laquelle sont formés plusieurs garnissages discrets, qui sont stabilisés et qui y sont collés, et le substrat est divisé pour séparer des autres chaque garnissage et sa partie de substrat associée.

4. Procédé selon l'une quelconque des revendications précédentes, caractérisé en ce qu'il s'exerce une force pression sur un côté du substrat et une faible pression sur l'autre côté, le matériau finement divisé étant introduit sur le côté à forte pression, et le gabarit (24, 106, 314) étant disposé sur le côté à forte pression du substrat.

5. Procédé selon l'une quelconque des revendications 1 à 3, caractérisé en ce qu'il s'exerce une forte pression sur un côté du substrat et une faible pression sur l'autre côté, le matériau finement divisé étant introduit sur le côté à forte pression, et le gabarit (312, 402) étant disposé sur le côté à faible pression du substrat.

6. Procédé selon l'une quelconque des revendications précédentes, caractérisé en ce que le substrat est supporté par un élément de support perméable aux fluides (35) pendant qu'on y forme le garnissage et pendant que le garnissage est stabilisé et y est collé.

7. Procédé selon la revendication 3, caractérisé en ce que le substrat est une bande qui est tirée depuis un rouleau de cette dernière (132, 102, 334), et les garnissages sont formés sur celle-ci, et ils sont stabilisés et y sont collés, d'une manière continue.

8. Procédé selon l'une quelconque des revendications précédentes, caractérisé en ce que le garnissage est stabilisé et collé au substrat par l'application d'un liant sur lui.

9. Procédé selon la revendication 8, caractérisé en ce que l'on chauffe le garnissage et substrat.

10. Procédé selon la revendication 2, caractérisé en ce qu'il comprend l'étape consistant à mouler le garnissage et sa partie associée du substrat pour avoir une configuration souhaitée avant séparation.

11. Procédé salon la revendication 10, caractérisé en ce que le garnissage et la partie de substrat associée sont moulés avant la stabilisation et le collage.

12. Procédé selon la revendication 10, caractérisé en ce que le garnissage et la partie de substrat associée sont moulés pendant la stabilisation et le collage.

13. Procédé selon la revendication 12, caractérisé en ce que le garnissage est stabilisé et collé au substrat au moyen d'un liant durcissable à la chaleur, et le garnissage et la partie du substrat associée sont moulés après application du liant sur eux, mais avant qu'il ne durcisse.

14. Procédé selon la revendication 1, caractérisé en ce qu'il comprend l'étape consistant à stratifier le garnissage et le substrat à un support imperméable aux liquides (42, 244), de sorte que le garnissage soit pris en sandwich encre le substrat et le support.

15. Procédé selon la revendication 1, caractérisé en ce que le matériau finement divisé est fibreux.

16. Procédé selon la revendication 1, caractérisé en ce que le substrat est un tissu non-tissé.

17. Appareil (200, 310, 406) pour fabriquer un produit composite ayant un garnissage discret (38, 70, 342) constitué d'un matériau finement divisé, sur un substrat perméable aux fluides (30, 100, 142, 336), ledit appareil comprenant des moyens de formation (20, 322) permettant de former le garnissage sur le substrat, lesdits moyens de formation comprenant des moyens de production de pression différentielle (27, 28, 120, 308) permettant de produire une pression différentielle à travers le substrat ; un gabarit (24, 106, 314, 402) permettant de définir sur le substrat la zone souhaitée à travers laquelle peut passer un fluide de transport ; et des moyens d'introduction (16, 18, 25, 125, 150, 154, 324) disposés sur un côté du substrat qui est, en cours d'utilisation, à une plus forte pression, permettant d'introduire le matériau finement divisé, en cours d'utilisation, dans un courant du fluide de transport afin d'être déposé sur le substrat dans la zone définie, caractérisé en ce que ledit appareil comprend aussi des moyens de stabilisation et de collage (200, 340, 344, 346) permettant de stabiliser le garnissage en collant au moins une partie de son matériau intérieurement et sur le substrat, lesdits moyens de stabilisation et de collage comprenant
(a) des moyens permettant d'introduire des particules thermoplastiques dans le matériau finement divisé, et des moyens (146) permettant de chauffer le garnissage et le substrat, ou
(b) des moyens (202, 340) permettant d'appliquer un liant sous forme de mousse au-dessus du garnissage, et des moyens (34, 130, 344) permettant de produire une pression différentielle au travers du garnissage et du substrat pour aspirer le liant dans le garnissage et le substrat.

18. Appareil selon la revendication 17, dans lequel le substrat est plus grand que le garnissage, caractérisé en ce qu'il comprend aussi des moyens de séparation permettant de séparer (58) du reste du substrat la portion du substrat à laquelle le garnissage est collé.

19. Appareil selon la revendication 18, caractérisé en ce qu'il comprend des moyens d'amenée de substrat (32, 103, 334) permettant d'amener un substrat de type feuille jusqu'aux moyens de formation, et dans lequel les moyens de formation forment, en cours d'utilisation, plusieurs garnissages discrets sur le substrat ; les moyens de stabilisation et de collage stabilisent tous les garnissages et les collent sur le substrat ; et les moyens de séparation divisent le substrat pour séparer des autres chaque garnissage et sa portion associée du substrat.

20. Appareil selon la revendication 18, caractérisé en ce que les moyens de séparation divisent le substrat, en cours d'utilisation, à une distance prédéterminée du bord du garnissage, pour réaliser une bordure autour de celui-ci.

21. Appareil selon l'une quelconque des revendications 17 à 20, caractérisé en ce que le gabarit (24, 106) et les moyens d'introduction (16, 18, 25, 125, 150, 152, 154) sont disposés du même côté du substrat.

22. Appareil selon l'une quelconque des revendications 17 à 20, caractérisé en ce que le gabarit (312, 402) et les moyens d'introduction (324) sont disposés sur les côtés opposés du substrat.

23. Appareil selon l'une quelconque des revendications 17 à 22, caractérisé en ce qu'il comprend un moyen de support perméable au fluide (35) permettant de supporter le substrat.

24. Appareil selon la revendication 19, caractérisé en ce que les moyens de formation et les moyens de stabilisation et de collage fonctionnent en continue pour former les garnissages sur une bande, les stabiliser sur celle-ci, puis les y coller, d'une manière continue, les moyens d'amenés de substrat amenant, en cours d'utilisation, la bande depuis un rouleau de cette dernière.

25. Appareil selon l'une quelconque des revendications 17 à 24, caractérisé en ce que les moyens de stabilisation et de collage comprennent un moyen de chauffage (208, 346) permettant de durcir un liant durcissable à le chaleur.

26. Appareil selon l'une quelconque des revendications 17 à 25, caractérisé en ce qu'il comprend des moyens de moulage (56, 222) permettant de mouler le garnissage et sa partie de substrat associée dans une configuration souhaitée.

27. Appareil selon l'une quelconque des revendications 17 à 26, caractérisé en ce qu'il comprend des moyens de stratification (46, 240) pour stratifier le garnissage et le substrat à un support imperméable aux liquides (42, 244), de sorte que le garnissage est pris en sandwich entre le substrat et le support.

28. Appareil selon l'une quelconque des revendications 17 à 27, caractérisé en ce que le matériau finement divisé est fibreux.

29. Appareil selon l'une quelconque des revendications 17 à 28, caractérisé en ce que le substrat est un tissu non-tissé.
